# EUROPÄISCHE PATENTSCHRIFT

(11) **EP 1 146 824 B1**
(45) Veröffentlichungstag und Bekanntmachung des Hinweises auf die Patenterteilung: **15.06.2005**
(21) Anmeldenummer: 99964673.0
(22) Anmeldetag: 24.12.1999
(51) Int. Cl.: A61B 17/122

(54) **MAGAZIN ZUR AUFNAHME VON CLIPS**
MAGAZINE FOR HOLDING CLIPS
MAGASIN POUR LOGER DES CLIPS

(30) Priorität: 30.01.1999 DE 19903752
(43) Veröffentlichungstag der Anmeldung: 24.10.2001
(73) Patentinhaber: Aesculap AG & Co. KG, 78532 Tuttlingen (DE)
(72) Erfinder: WEISSHAUPT, Dieter, D-78194 Immendingen (DE)
(74) Vertreter: Böhme, Ulrich, Dr. Dipl.-Phys.
(86) Internationale Anmeldenummer: PCT/EP1999/010370
(87) Internationale Veröffentlichungsnummer: WO 2000/044290

(56) Entgegenhaltungen:
- WO-A-91/04925
- WO-A-98/05260
- US-A- 5 046 611

## Beschreibung

Die Erfindung betrifft ein Magazin zur Aufnahme U-förmiger, einen Steg und zwei davon abstehende Arme aufweisender Clips mit einer an der Unterseite des Steges anliegenden Stütze und mit zumindest einseitig seitlich an dem Steg anliegenden, elastisch quer zur Ebene des Clips von diesem Steg entfernbaren Klemmgliedern.

Ein solches Magazin ist beispielsweise aus der WO 98/05260 bekannt. Die in einem solchen Magazin aufgenommenen Clips sind teilweise sehr klein, beispielsweise liegen die Abmessungen in der Größenordnung von 1,5 mm, so daß die Handhabung dieser Clips außerordentlich schwierig ist. Bei den bekannten Magazinen werden die Clips in den Magazinen so gehalten, daß ein zangenförmiges Anlegeinstrument beim Eintauchen in die Magazine die Clips erfassen und dann aus dem Magazin herausziehen kann. Dabei ist bei den bekannten Magazinen nachteilig, daß die Clips seitlich durch elastisch federnde Klemmelemente fixiert werden, die nur im mittleren Bereich des Steges anliegen und die beim Einschieben eines Anlegewerkzeuges vom Clip entfernt werden, so daß die seitliche Fixierung der Clips beim Einschieben des Anlegewerkzeuges aufgehoben wird. Es kann daher beim Einschieben des Anlegewerkzeuges zu einer Verkippung oder gar Verklemmung des Clips im Magazin kommen, und dann ist es nicht mehr möglich, den Clip in der vorgesehenen Weise in das Clipanlegeinstrument einzuführen.

Es ist Aufgabe der Erfindung, ein gattungsgemäßes Magazin derart auszubilden, daß der Clip im Magazin in jeder Phase vollständig eindeutig und kippfrei gehalten ist, insbesondere auch beim Einführen eines Anlegewerkzeuges in das Magazin.

Diese Aufgabe wird bei einem Magazin der eingangs beschriebenen Art erfindungsgemäß dadurch gelöst, daß zusätzliche, zumindest einseitig seitlich an den Armen anliegende, elastisch quer zur Ebene des Clips von diesen Armen entfernbare Klemmglieder vorgesehen sind, die von den am Steg anliegenden Klemmgliedern unabhängig elastisch gegen die Arme angedrückt sind.

Damit wird der Clip im Magazin durch elastische Klemmglieder gehalten, die sowohl im Bereich des Steges als auch im Bereich der Arme am Clip anliegen und diesen somit an unterschiedlichen Stellen halten. Da die Klemmglieder unabhängig voneinander elastisch von den Armen entfernbar sind, bleiben die an den Armen anliegenden Klemmglieder auch dann in ihrer Klemmposition, wenn ein Anlegewerkzeug in das Magazin eingeschoben wird und dabei die Klemmglieder von dem Clip entfernt, die im Bereich des Steges an diesem anliegen. Der Clip ist auch in dieser Phase durch die an den Armen anliegenden Klemmglieder sicher gehalten. Beim weiteren Einschieben des Anlegeinstrumentes werden zwar diese Klemmglieder, die normalerweise an den Armen anliegen, auch elastisch abgebogen, dann ist aber der Clip bereits sicher in dem Anlegeinstrument geführt und kann nicht mehr verkippt werden.

Bei einer ersten bevorzugten Ausführungsform kann vorgesehen sein, daß das Magazin nur an einer Seite des Clips elastisch von den Armen entfernbare Klemmglieder aufweist und auf der gegenüberliegenden Seite eine feststehende Anlagefläche für den Clip. Bei einer solchen Konstruktion wird der Clip durch die federnden Klemmelemente gegen eine stationäre Anlagefläche gedrückt und dadurch fixiert. Bei einer anderen Ausführungsform kann vorgesehen sein, daß auf beiden Seiten des Clips seitlich an diesem anliegende, von ihm entfernbare Klemmglieder vorgesehen sind, die die Teile des Clips paarweise zwischen sich halten. Bei dieser Ausführungsform liegen die federnden Klemmglieder an gegenüberliegenden Seiten am Clip an, so daß dieser zwischen diesen federnden Klemmgliedern exakt geführt und zentriert wird. Dabei erfolgt eine Führung durch paarweise Anlage von auf gegenüberliegenden Seiten angeordnete Klemmglieder in unterschiedlichen Höhen des Clips, so daß insgesamt eine optimale Halterung des Clips im Magazin zu erzielen ist.

Günstig ist es, wenn die Klemmglieder Federarme aufweisen, die an ihrem freien Ende an den Steg bzw. die Arme anlegbare Andruckflächen tragen. Diese Andruckflächen können insbesondere ballig vorstehen.

Bei einer bevorzugten Ausführungsform ist vorgesehen, daß am Steg an dessen beiden Endbereichen jeweils eine Andruckfläche elastisch anliegt, damit wird der Steg an zwei Stellen zwischen den Klemmgliedern gehalten und ist dadurch gegen eine Verschwenkung gesichert.

Die beiden Andruckflächen können an einem Steg angeordnet sein, der von zwei Federarmen getragen wird, die zwischen sich einen Abstand einhalten. Man erhält somit ein U-förmiges Andruckelement, an dem zwei Andruckflächen ausgebildet sind.

Günstig ist es, wenn im Zwischenraum zwischen den Federarmen die zusätzlichen Klemmglieder angeordnet sind.

Dabei können die Klemmglieder, die am Steg und den Armen anliegen, sich in einer parallel zur Clipebene verlaufenden Ebene befinden, so daß eine optimale Ausnützung des zur Verfügung stehenden Platzes gegeben ist.

Bei einer besonders bevorzugten Ausführungsform ist vorgesehen, daß das Magazin einen U-förmigen Grundkörper aufweist mit einem Steg und mit zwei von diesem abstehenden Schenkeln, daß am Steg die zwischen die Schenkel eintauchende Stütze angeordnet ist und daß die Klemmglieder zwischen den Schenkeln und der Stütze angeordnet sind.

Dabei ist es vorteilhaft, wenn die Klemmglieder plattenförmig ausgebildet sind und dicht nebeneinanderliegen, so daß sie eine mehrteilige Seitenwand für den Zwischenraum zwischen den Schenkeln und der Stütze ausbilden. Man erhält somit einen allseits geschlossenen Aufnahmeraum im Inneren des Magazins, der an den Schmalseiten durch die Schenkel und an den Breitseiten durch die plattenförmig ausgebildeten Klemmglieder begrenzt wird.

Zusätzlich kann vorgesehen sein, daß die Schenkel an ihren freien Enden durch parallele Stege miteinander verbunden sind, die zwischen sich eine Einführöffnung für einen Clip und für ein Anlegewerkzeug ausbilden. Dadurch wird die Festigkeit des Magazins erhöht.

Es ist besonders vorteilhaft, wenn der Grundkörper, die Stütze, die Klemmglieder und gegebenenfalls die die Schenkel des Grundkörpers verbindenden Stege einteilig aus Kunststoff ausgebildet sind.

Mehrere dieser gleichartigen Magazine können übereinander angeordnet sein und miteinander stapelartig verbunden sein, so daß in einem solchen Magazinstapel eine größere Anzahl von Clips aufgenommen werden kann.

Bei einer anderen bevorzugten Ausführungsform ist vorgesehen, daß mehrere gleichartige Magazine nebeneinander in einer Ebene angeordnet sind.

Beispielsweise können die Magazine parallel zueinander in Form eines Streifens angeordnet sein.

Bei einer anderen bevorzugten Ausführungsform sind die Magazine am Außenrand einer Scheibe angeordnet, insbesondere einer kreisförmigen Scheibe, und ihre Einführseiten sind voneinander weggerichtet.

Die nachfolgende Beschreibung bevorzugter Ausführungsformen der Erfindung dient im Zusammenhang mit der Zeichnung der näheren Erläuterung. Es zeigen:
- Figur 1:: einen Cliphalter mit stapelförmig übereinander angeordneten Magazinen und ein Anlegewerkzeug beim Einführen in eines der Magazine;
- Figur 2: ein streifenförmiger Halter mit mehreren nebeneinander angeordneten Magazinen;
- Figur 3:: ein scheibenförmiger Halter mit mehreren längs des Scheibenumfanges angeordneten Magazinen;
- Figur 4:: eine Schnittansicht längs Linie 4-4 in Figur 1;
- Figur 5:: eine Schnittansicht längs Linie 5-5 in Figur 4;
- Figur 6:: eine Schnittansicht längs Linie 6-6 in Figur 4;
- Figur 7:: eine Schnittansicht eines Magazins längs Linie 4-4 in Figur 1 mit einem teilweise eingeschobenen Anlegewerkzeug;
- Figur 8:: eine Schnittansicht längs Linie 8-8 in Figur 7 und
- Figur 9:: eine Ansicht ähnlich Figur 8 mit vollständig eingeschobenem Anlegewerkzeug.

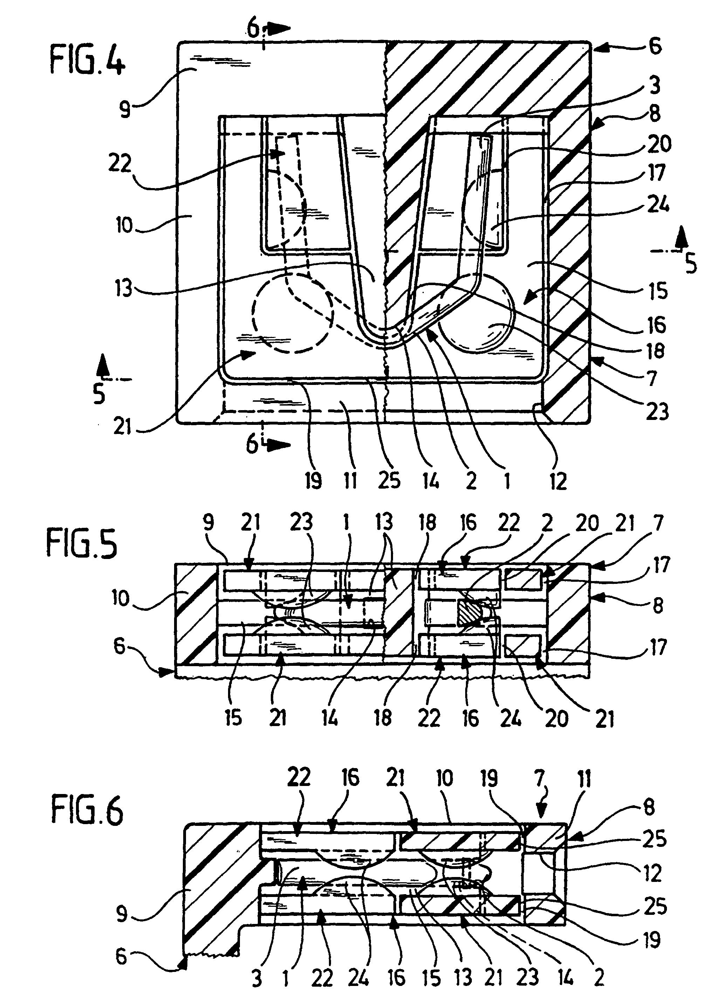

Die in der Zeichnung dargestellten Halter dienen der Aufnahme von U-förmigen Clips 1, bzw. hämostatischen Clips, die an Blutgefäßen angelegt und zusammengedrückt werden, um die Blutgefäße zu unterbinden. Derartige Clips 1 umfassen einen Steg 2 sowie zwei von diesem endseitig abstehende Arme 3, die in derselben Ebene liegen wie der Steg 2. In dem in der Zeichnung dargestellten Ausführungsbeispiel ist der Steg 2 in der Mitte abgewinkelt, und die Arme 3 stehen so vom Steg 2 ab, daß sie zu ihrem freien Ende hin geringfügig divergieren (Figur 4). Die Clips 1 bestehen üblicherweise aus einem körperverträglichen Metall, beispielsweise aus Edelstahl oder Titan, und können dauerhaft derart verformt werden, daß die beiden Arme 3 einander angenähert werden und dabei ein abzubindendes Gefäß zwischen sich aufnehmen.

Das Anlegen eines derartigen Clips 1 erfolgt beispielsweise durch ein Anlegewerkzeug mit zwei gegeneinander bewegbaren Branchen 4, die an ihrem freien Ende an den einander zugewandten Seiten parallel zu den Branchen 4 verlaufende, zur gegenüberliegende Branche jeweils offene Längsnuten 5 tragen. Die Branchen 4 werden bei einem derartigen Anlegewerkzeug so von der Stegseite her über den Clip 1 geschoben, daß die Arme 3 in die Längsnuten 5 eingreifen und geringfügig gegeneinander gedrückt werden, so daß ein derartiger Clip 1 zwischen den Branchen 4 festgehalten wird. Durch Annähern der Branchen 4, beispielsweise mittels eines in der Zeichnung nicht dargestellten Griffteils am Anlegewerkzeug, kann der Clip 1 in der beschriebenen Weise zusammengedrückt werden.

Zur Aufbewahrung einer größeren Anzahl derartiger Clips 1 werden Halter 6 verwendet, die jeweils eine größere Anzahl von gleich aufgebauten Magazinen 7 aufweisen. Jedes dieser Magazine 7 (Figuren 4 bis 9) umfaßt einen U-förmigen Grundkörper 8 mit einem geradlinigen Steg 9 und an dessen Enden senkrecht von diesem abstehenden Schenkeln 10, die an ihren freien Enden durch zwei parallel zueinander verlaufende Stege 11 miteinander verbunden sind. Zwischen den beiden Stegen 11 ergibt sich eine Einführöffnung 12, die einen Zugang zu dem Innenraum des Grundkörpers bildet.

In der Mitte des Steges 9 steht von diesem eine Stütze 13 ab, die parallel zu den Schenkeln 10 verläuft und die weniger lang ist als diese, so daß sie im Abstand von den Stegen 11 endet. Diese Stütze 13 weist an ihrem oberen Ende eine Einlegenut 14 für den Mittelbereich des Steges 2 des Clips 1 auf, diese Einlegenut 14 verläuft in der Längsmittelebene des Grundkörpers 8. Die Stütze 13 erstreckt sich dabei im wesentlichen über die gesamte Dicke des Grundkörpers 8 und unterteilt somit den zwischen den Schenkeln 10 angeordneten Aufnahmeraum 15 des Grundkörpers 8 in zwei Teile.

Der Aufnahmeraum 15 wird durch zwei parallel zu den Stegen 11 verlaufende Seitenwände 16 abgeschlossen, die längs der Längskanten des Schenkels 10 mit diesem verbunden sind, die aber sonst keine Verbindung zum Grundkörper 8 eingehen ,d.h. zwischen diesen Seitenwänden 16 und den angrenzenden Schenkeln 10 sowie der Stütze 13 sind schmale Spalte 17 bzw. 18 vorgesehen, auch zwischen den Stegen 11 und den Seitenwänden 16 befindet sich ein derartiger Spalt 19.

Die Seitenwände 16 sind weiterhin durch einen Spalt 20 derart unterteilt, daß ein äußerer U-förmiger Seitenwandabschnitt 21 und zwei im Abstand zueinander angeordnete Seitenwandabschnitte 22 entstehen, die von dem U-förmigen Seitenwandabschnitt 21 umgeben werden und zwischen denen sich die Stütze 13 befindet (Figur 4).

Die Seitenwandabschnitte 21 und 22 können elastisch federnd aus ihrer Ebene nach außen gebogen werden, sie tragen an ihren einander zugewandten Innenseiten ballige Vorsprünge 23 bzw. 24, die einander jeweils gegenüberliegen. Dabei sind zwei derartige Vorsprünge 23 im Abstand zueinander und der freien Kante 25 des Seitenwandabschnittes 21 benachbart vorgesehen, während jeder der Seitenwandabschnitte 22 jeweils einen derartigen Vorsprung 24 an seinem freien Ende trägt. Sowohl der U-förmige Seitenwandabschnitt als auch die beiden Seitenwandabschnitte 22 bilden somit Federelemente aus, die an ihrer Innenseite die Vorsprünge 23 bzw. 24 tragen.

Das gesamte bisher beschriebene Magazin 7 wird vorzugsweise einteilig aus Kunststoff hergestellt und bildet einen Aufnahmeraum 15 aus für einen Clip 1.

Dieser wird so in den Aufnahmeraum 15 eingeschoben, daß sein Steg 2 mit dem abgewinkelten Mittelbereich in die Einlegenut 14 der Stütze 13 eingreift, während die beiden Arme 3 des Clips 1 in die beiden durch die Stütze 13 voneinander getrennten Teile des Aufnahmeraumes 15 eintaucht. Die Endbereiche des Steges 2 kommen dabei zwischen die Vorsprünge 23 des Seitenwandabschnittes 21 zu liegen, die Arme 3 zwischen die Vorsprünge 24 von jeweils einander gegenüberliegenden Seitenwandabschnitten 22. Die Vorsprünge 23 und 24 bilden also Klemmglieder, die den Clip 1 im eingeschobenen Zustand in der eingeschobenen Lage exakt positionieren und festhalten, wie dies aus den Darstellungen der Figuren 4 bis 6 deutlich wird.

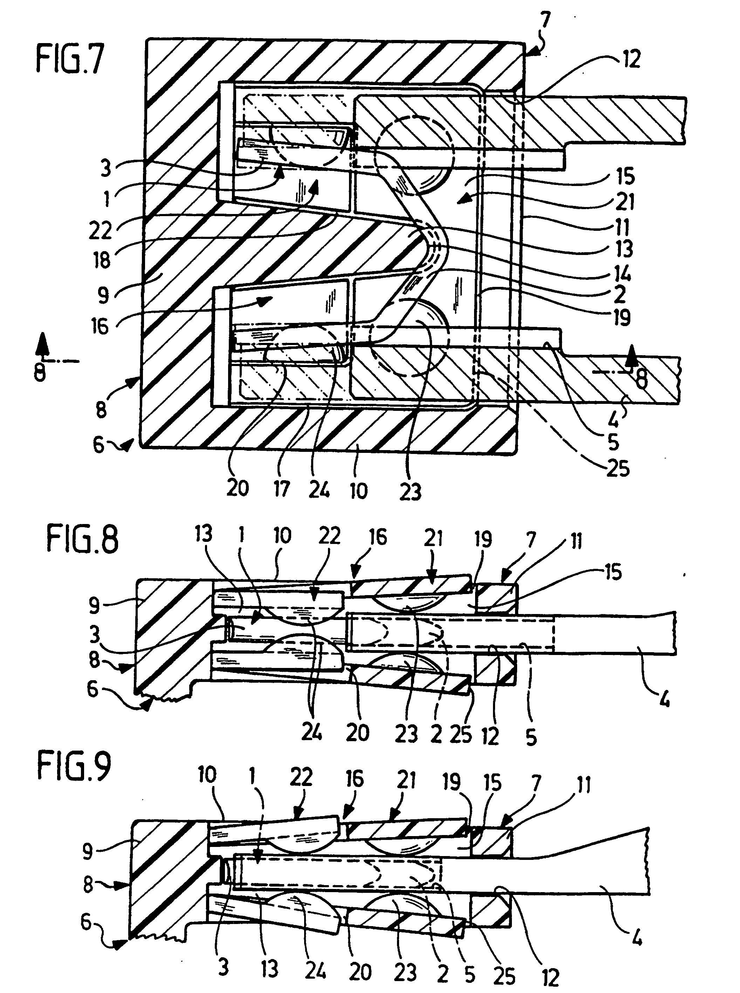

Um einen Clip 1 aus einem solchen Magazin 7 zu entnehmen, werden die beiden Branchen 4 des Anlegewerkzeuges durch die Einführöffnung 12 in den Aufnahmeraum 15 eingeschoben, so daß sie über die Arme 3 des Clips 1 gleiten (Figur 7). Bei Beginn des Einschiebevorganges der Branchen 4 kommen diese an den Vorsprüngen 23 des Seitenwandabschnittes 21 zur Anlage und biegen dadurch die Seitenwandabschnitte 21 elastisch nach außen, d.h. die Anlage der Vorsprünge 23 am Clip 1 wird aufgehoben. Der Clip wird aber weiterhin einmal durch die Längsnuten 5 der Branchen 4 und zum anderen durch die Vorsprünge 24 an den Seitenwandabschnitten 22 in seiner Lage fixiert, so daß beim weiteren Vorschieben der Branchen 4 die exakte Positionierung des Clips aufrechterhalten wird. Bei diesem weiteren Einschieben drücken schließlich die Branchen 4 auch die Vorsprünge 24 und die sie tragenden Seitenwandabschnitte 22 nach außen (Figur 9), so daß der Clip nun ausschließlich in den Längsnuten 5 der Branchen 4 und in der Einlegenut 14 der Stütze 13 gehalten wird. Sobald die Branchen 4 vollständig eingeschoben sind, können sie zusammen mit dem Clip 1 aus dem Aufnahmeraum 15 herausgezogen werden, da die Vorsprünge 23 und 24 durch die Branchen 4 nach außen gebogen sind, können sie den Clip 1 nicht mehr festhalten, dieser wir allein durch die Elastizität der an der Innenseite der Längsnuten 5 anliegenden Arme 3 zwischen den Branchen 4 gehalten.

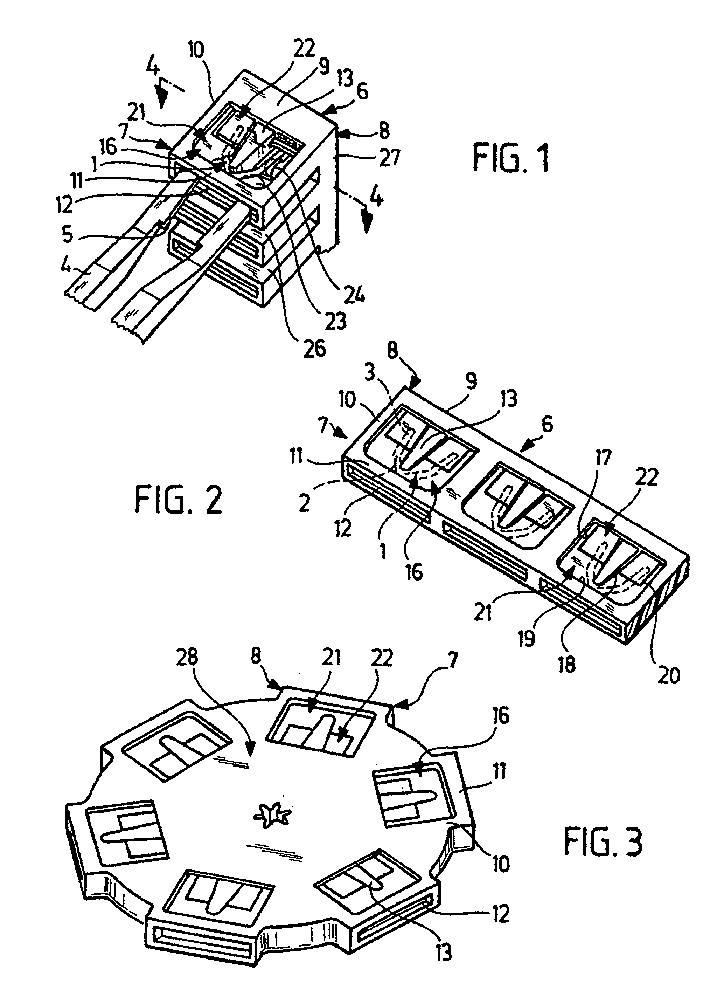

Bei dem Ausführungsbeispiel der Figur 1 sind mehrere gleichartige Magazine 7 stapelartig übereinander angeordnet, wobei zwischen benachbarten Magazinen 7 ein Zwischenraum 26 eingehalten ist, die Stege 9 der Grundkörper 8 sind zu einer Grundplatte 27 verbunden, so daß in einem solchen stapelförmigen Halter eine größere Anzahl von Magazinen parallel zueinander angeordnet sind, von denen jedes einen Clip 1 aufnehmen kann.

Bei dem Ausführungsbeispiel der Figur 2 sind mehrere gleichartige Magazine 17 streifenförmig in einer Ebene nebeneinander angeordnet, wobei die Grundkörper 8 der nebeneinanderliegenden Magazine 7 einen gemeinsamen Steg 9 aufweisen und wobei auch die Schenkel 10 benachbarter Grundkörper 8 jeweils zwei benachbarten Grundkörpern 8 gemeinsam sind. Die Einführöffnungen 12 liegen dabei alle auf einer Seite des streifenförmigen Halters 6.

Bei dem Ausführungsbeispiel der Figur 3 sind gleichartig ausgestaltete Magazine 7 in einer kreisförmigen Scheibe 28 eingearbeitet, welche die Grundkörper 8 der Magazine 7 aufnimmt, die alle einstückig ausgebildet sind. Die Orientierung ist dabei so gewählt, daß die Einführöffnungen 12 der längs des Umfanges verteilten Magazine 7 voneinander weggerichtet sind, also radial nach außen.

## Patentansprüche

1. Magazin zur Aufnahme U-förmiger, einen Steg (2) und zwei davon abstehende Arme (3) aufweisender Clips (1) mit einer an der Unterseite des Steges (2) anliegenden Stütze (13) und mit zumindest einseitig seitlich an dem Steg (2) anliegenden, elastisch quer zur Ebene des Clips (1) von diesem Steg (2) entfernbaren Klemmgliedern, **dadurch gekennzeichnet, daß** zusätzliche, zumindest einseitig seitlich an den Armen (3) anliegende, elastisch quer zur Ebene des Clips (1) von diesen Armen (3) entfernbare Klemmglieder (22, 24) vorgesehen sind, die von den am Steg (2) anliegenden Klemmgliedern (21, 23) unabhängig elastisch gegen die Arme angedrückt (3) sind.

2. Magazin nach Anspruch 1, **dadurch gekennzeichnet, daß** das Magazin nur an einer Seite des Clips elastisch von den Armen entfernbare Klemmglieder (21, 22, 23, 24) aufweist und auf der gegenüberliegenden Seite eine feststehende Anlagefläche für den Clip (1).

3. Magazin nach Anspruch 1, **dadurch gekennzeichnet, daß** auf beiden Seiten des Clips seitlich an diesem anliegende, von ihm entfernbare Klemmglieder (21, 22, 23, 24) vorgesehen sind, die die Teile des Clips (1) paarweise zwischen sich halten.

4. Magazin nach einem der voranstehenden Ansprüche, **dadurch gekennzeichnet, daß** die Klemmglieder Federarme (21, 22) aufweisen, die an ihrem freien Ende an den Steg (2) bzw. die Arme (3) anlegbare Andruckflächen (23 bzw. 24) tragen.

5. Magazin nach Anspruch 4, **dadurch gekennzeichnet, daß** die Andruckflächen (23, 24) ballig vorstehen.

6. Magazin nach einem der Ansprüche 4 oder 5, **dadurch gekennzeichnet, daß** am Steg (2) an dessen beiden Endbereichen jeweils eine Andruckfläche (23) elastisch anliegt.

7. Magazin nach Anspruch 6, **dadurch gekennzeichnet, daß** die beiden Andruckflächen (23) an einem Steg angeordnet sind, der von zwei Federarmen getragen wird, die zwischen sich einen Abstand einhalten.

8. Magazin nach Anspruch 7, **dadurch gekennzeichnet, daß** im Zwischenraum zwischen den Federarmen die zusätzlichen Klemmglieder (22, 24) angeordnet sind.

9. Magazin nach einem der voranstehenden Ansprüche, **dadurch gekennzeichnet, daß** die Klemmglieder (21, 22), die am Steg (2) bzw. den Armen (3) anliegen, sich in einer parallel zur Clipebene verlaufenden Ebene befinden.

10. Magazin nach einem der voranstehenden Ansprüche, **dadurch gekennzeichnet, daß** es einen U-förmigen Grundkörper (8) aufweist mit einem Steg (9) und zwei von diesem abstehenden Schenkeln (10), daß am Steg (9) die zwischen die Schenkel (10) eintauchende Stütze (13) angeordnet ist und daß die Klemmglieder (21, 23; 22, 24) zwischen den Schenkeln (10) und der Stütze (13) angeordnet sind.

11. Magazin nach Anspruch 10, **dadurch gekennzeichnet, daß** die Klemmglieder (21, 22) plattenförmig ausgebildet sind und dicht nebeneinanderliegen, so daß sie eine mehrteilige Seitenwand (16) für den Zwischenraum (15) zwischen den Schenkeln (10) und der Stütze (13) ausbilden.

12. Magazin nach einem der Ansprüche 10 oder 11, **dadurch gekennzeichnet, daß** die Schenkel (10) an ihren freien Enden durch parallele Stege (11) miteinander verbunden sind, die zwischen sich eine Einführöffnung (12) für einen Clip (1) und für ein Anlegewerkzeug (4) ausbilden.

13. Magazin nach einem der Ansprüche 10 bis 12, **dadurch gekennzeichnet, daß** der Grundkörper (8), die Stütze (13), die Klemmglieder (21, 23; 22, 24) und gegebenenfalls die die Schenkel (10) des Grundkörpers (8) verbindenden Stege (11) einteilig aus Kunststoff ausgebildet sind.

14. Magazin nach einem der voranstehenden Ansprüche, **dadurch gekennzeichnet, daß** mehrere gleichartige Magazine (7) übereinander angeordnet und miteinander stapelartig verbunden sind.

15. Magazin nach einem der Ansprüche 1 bis 13, **dadurch gekennzeichnet, daß** mehrere gleichartige Magazine (7) nebeneinander in einer Ebene angeordnet sind.

16. Magazin nach Anspruch 15, **dadurch gekennzeichnet, daß** die Magazine (7) parallel zueinander in Form eines Streifens angeordnet sind.

17. Magazin nach Anspruch 15, **dadurch gekennzeichnet, daß** die Magazine (7) am Außenrand einer Scheibe (28) angeordnet sind und daß ihre Einführöffnungen (12) voneinander weggerichtet sind.

## Claims

1. Cartridge for receiving U-shaped clips (1) having a web (2) and two arms (3) projecting therefrom, with a support (13) abutting the underside of the web (2) and with clamping members, which abut the web (2) laterally at least on one side and are elastically removable from this web (2) transversely to the plane of the clip (2), **characterised in that** additional clamping members (22, 24) are provided, which abut the arms (3) laterally at least on one side and are elastically removable from these arms (3) transversely to the plane of the clip (1), and which are pressed elastically against the arms (3) independently of the clamping members (21, 23) abutting the web (2).

2. Cartridge according to Claim 1, **characterised in that** the cartridge has clamping members (21, 22, 23, 24), which are elastically removable from the arms on only one side of the clip, and a fixed contact surface for the clip (1) on the opposite side.

3. Cartridge according to Claim 1, **characterised in that** clamping members (21, 22, 23, 24), which abut the clip and are removable therefrom, are provided on both sides of the clip, said clamping members holding the parts of the clip (1) between them in pairs.

4. Cartridge according to any one of the preceding claims, **characterised in that** the clamping members have spring arms (21, 22), which at their free end bear pressure surfaces (23 or 24) which may be laid against the web (2) or the arms (3).

5. Cartridge according to Claim 4, **characterised in that** the pressure surfaces (23, 24) protrude in the shape of a ball.

6. Cartridge according to Claim 4 or 5, **characterised in that** a respective pressure surface (23) elastically abuts the web (2) at its two end regions.

7. Cartridge according to Claim 6, **characterised in that** the two pressure surfaces (23) are disposed on a web which is supported by two spring arms which maintain a distance between them.

8. Cartridge according to Claim 7, **characterised in that** the additional clamping members (22, 24) are disposed in the interstice between the spring arms.

9. Cartridge according to any one of the preceding claims, **characterised in that** the clamping members (21, 22) abutting the web (2) or the arms (3) are located in a plane running parallel to the plane of the clip.

10. Cartridge according to any one of the preceding claims, **characterised in that** it has a U-shaped base body (8) with a web (9) and two legs (10) projecting from these, that the support (13) penetrating between the legs (10) is disposed on the web (9), and that the clamping members (21, 23; 22, 24) are disposed between the legs (10) and the support (13).

11. Cartridge according to Claim 10, **characterised in that** the clamping members (21, 22) are in the form of plates and lie close together, so that they form a multiple-part side wall (16) for the interstice (15) between the legs (10) and the support (13).

12. Cartridge according to Claim 10 or 11, **characterised in that** the legs (10) are connected to one another at their free ends by parallel webs (11), which between them form an insertion opening (12) for a clip (1) and for an application tool (4).

13. Cartridge according to any one of claims 10 to 12, **characterised in that** the base body (8), the support (13), the clamping members (21, 23; 22, 24) and possibly the webs (11) connecting the legs (10) of the base body (8) are made of plastic in one piece.

14. Cartridge according to any one of the preceding claims, **characterised in that** several cartridges (7) of the same type are arranged one above the other and joined to one another in the manner of a stack.

15. Cartridge according to any one of claims 1 to 13, **characterised in that** several cartridges (7) of the same type are arranged next to one another in one plane.

16. Cartridge according to Claim 15, **characterised in that** the cartridges (7) are arranged parallel to one another in the form of a strip.

17. Cartridge according to Claim 15, **characterised in that** the cartridges (7) are arranged on the outer edge of a disc (28) and that their insertion openings (12) are directed away from one another.

## Revendications

1. Magasin pour loger des clips (1) en forme de U, comportant une nervure (2) et deux bras (3) partant de celle-ci, avec un montant (13) s'appuyant sur la face inférieure de la nervure (2) et avec des éléments de serrage s'appuyant au moins d'un côté latéralement sur la nervure (2), démontables de cette nervure (2) de manière élastique perpendiculairement au plan du clip (1), **caractérisé en ce qu'**il est prévu des éléments de serrage supplémentaires (22, 24) s'appuyant au moins d'un côté latéralement sur les bras (3), démontables de ces bras (3) de manière élastique perpendiculairement au plan du clip (1), qui sont pressés contre les bras (3) de manière élastique, indépendamment des éléments de serrage (21, 23) s'appuyant sur la nervure (2).

2. Magasin selon la revendication 1, **caractérisé en ce que** le magasin comporte des éléments de serrage (21, 22, 23, 24) démontables des bras de manière élastique seulement d'un côté du clip et, du côté opposé, une surface d'appui fixe pour le clip (1).

3. Magasin selon la revendication 1, **caractérisé en ce qu'**il est prévu des deux côtés du clip des éléments de serrage (21, 22, 23, 24) s'appuyant latéralement sur celui-ci, démontables de celui-ci, qui maintiennent entre elles par paires les parties du clip (1).

4. Magasin selon l'une quelconque des revendications précédentes, **caractérisé en ce que** les éléments de serrage comportent des bras à ressort (21, 22) qui, à leur extrémité libre, portent des surfaces de pression (23 ou 24) applicables sur la nervure (2) ou sur les bras (3).

5. Magasin selon la revendication 4, **caractérisé en ce que** les surfaces de pression (23 ou 24) font saillie de manière convexe.

6. Magasin selon l'une quelconque des revendications 4 ou 5, **caractérisé en ce qu'**une surface de pression (23) s'appuie élastiquement sur la nervure (2) aux deux zones d'extrémité de celle-ci.

7. Magasin selon la revendication 6, **caractérisé en ce que** les deux surfaces de pression (23) sont disposées sur une nervure qui est portée par deux bras à ressort qui maintiennent une distance entre eux.

8. Magasin selon la revendication 7, **caractérisé en ce que** les éléments de serrage supplémentaires (22, 24) sont disposés dans l'espace intermédiaire entre les bras à ressort.

9. Magasin selon l'une quelconque des revendications précédentes, **caractérisé en ce que** les éléments de serrage (21, 22) qui s'appuient sur la nervure (2) ou sur les bras (3) se trouvent dans un plan s'étendant parallèlement au plan des clips.

10. Magasin selon l'une quelconque des revendications précédentes, **caractérisé en ce qu'**il comporte un corps de base en forme de U (8) avec une nervure (9) et deux branches (10) partant de celle-ci, **en ce que** le montant (13) plongeant entre les branches (10) est disposé sur la nervure (9), et **en ce que** les éléments de serrage (21, 22, 23, 24) sont disposés entre les branches (10) et le montant (13).

11. Magasin selon la revendication 10, **caractérisé en ce que** les éléments de serrage (21, 22) sont configurés en forme de plaque et sont placés tout près les uns des autres, de sorte qu'ils forment une paroi latérale à plusieurs parties (16) pour l'espace intermédiaire (15) entre les branches (10) et le montant (13).

12. Magasin selon la revendication 10 ou 11, **caractérisé en ce que** les branches (10) sont reliées les unes aux autres à leurs extrémités libres par des nervures parallèles (11) qui forment entre elles une ouverture d'introduction (12) pour un clip (1) et pour un outil de mesure à pince (4).

13. Magasin selon l'une quelconque des revendications 10 à 12, **caractérisé en ce que** le corps de base (8), le montant (13), les éléments de serrage (21, 23 ; 22, 24) et éventuellement les nervures (11) reliant les branches (10) du corps de base (8) sont configurés en une seule partie constituée de matière plastique.

14. Magasin selon l'une quelconque des revendications précédentes, **caractérisé en ce que** plusieurs magasins similaires (7) sont disposés les uns sur les autres et sont reliés les uns aux autres à la manière d'un empilement.

15. Magasin selon l'une quelconque des revendications 1 à 13, **caractérisé en ce que** plusieurs magasins similaires (7) sont disposés les uns à côté des autres dans un plan.

16. Magasin selon la revendication 15, **caractérisé en ce que** les magasins (7) sont disposés parallèlement les aux autres sous forme d'une bande.

17. Magasin selon la revendication 15, **caractérisé en ce que** les magasins (7) sont disposés sur le bord extérieur d'un disque (28) et **en ce que** leurs ouvertures d'introduction (12) sont orientées à l'opposé les unes des autres.
